# EUROPÄISCHE PATENTSCHRIFT

(11) **EP 0 786 083 B1**
(45) Veröffentlichungstag und Bekanntmachung des Hinweises auf die Patenterteilung: **09.08.2000**
(21) Anmeldenummer: 95935420.0
(22) Anmeldetag: 30.09.1995
(51) Int. Cl.: G01N 33/50, C12Q 1/533

(54) **VERFAHREN ZUR DIAGNOSTISCHEN BEURTEILUNG UND VERLAUFSKONTROLLE SOWIE ARZNEIMITTEL ZUR THERAPIE VON SCHOCKZUSTÄNDEN BEIM MENSCHEN**
DIAGNOSIS AND MONITORING PROCESS, AND MEDICAMENTS FOR THE THERAPY OF STATES OF SHOCK IN HUMAN BEINGS
PROCEDE DE DIAGNOSTIC ET DE SURVEILLANCE ET MEDICAMENT PERMETTANT DE TRAITER DES ETATS DE CHOC CHEZ L'ETRE HUMAIN

(30) Priorität: 12.10.1994 DE 4436352
(43) Veröffentlichungstag der Anmeldung: 30.07.1997
(73) Patentinhaber: PAZ ARZNEIMITTEL- ENTWICKLUNGSGESELLSCHAFT MBH, 65933 Frankfurt (DE)
(72) Erfinder: BANG, Holger, D-91036 Erlangen (DE); BRUNE, Kay, D-91080 Marloffstein (DE); WENDEL, Albrecht, D-72070 Tübingen (DE); TIEGS, Gesa, D-78465 Konstanz (DE)
(74) Vertreter: Grussdorf, Jürgen, Dr.
(86) Internationale Anmeldenummer: EP9503881
(87) Internationale Veröffentlichungsnummer: WO9612184

(56) Entgegenhaltungen:
- EP-A- 0 285 603
- EP-A- 0 326 067
- WO-A-83/00930
- WO-A-92/14455

## Beschreibung

Gegenstand der vorliegenden Erfindung sind ein neues Verfahren zur diagnostischen Beurteilung und Verlaufskontrolle sowie ein Arzneimittel zur Therapie von Schockzuständen beim Menschen.

In den westlichen Industrienationen versterben jedes Jahr Tausende direkt an verschiedenen Schockformen oder den unmittelbaren Folgen des Schocks. Als Schock versteht man eine akut auftretende, durch Blutdruckabfall gekennzeichnete Fehlregulation der Durchblutung lebenswichtiger Organe. Aus dieser akut auftretenden Fehlregulation entwickeln sich häufig diffuse oder selektive Organschäden und ein protrahiertes Organversagen, das in ca. 10 bis 30 % der Falle zum Tode führt (vgl. z.B. W. Pschyrembel, Klin. Wörterbuch, de Gruyter Berlin, 1992). Derartige Schockreaktionen können auf der Basis von akuten und chronischen Infektionen, bei vorgeschädigten Patienten (postoperativ), Behandlung mit Zytostatika etc. oder auch ohne erkennbaren Anlaß, meist im Gefolge von im übrigen harmlosen bakteriellen Infektionen auftreten. Man unterscheidet anaphylaktische, bakterielle, septische, posttraumatische, cardiogene, hämorrhagische, hypovolämische, neurogene und toxische Schockformen. Ein gemeinsamer Nenner scheint in der Koexistenz eines veränderten Abwehrverhaltens des Körpers gegenüber häufig harmlosen Mikroben auf der Basis einer gleichzeitig oder kurz davor erfolgten traumatischen, chirurgischen, immunologischen, medikamentösen oder toxischen Vorschädigung des Organismus (vgl. z.B. Bochner, B.S. et al., N Engl J Med 1991, 3241 1785) zu bestehen. Ein einheitliches und gesichertes Therapieverfahren, insbesondere des posttraumatischen, postoperativen, toxischen oder septischen Schocks besteht bisher nicht (Barron, R.L., Clin Pharm 1993, 12, 829). Verläßliche Kriterien zur Beurteilung der Prognose und des Therapieerfolgs bei der Schockkrankheit fehlen. Spezifische, anerkannte und rational begründete Therapiekonzepte sind daher nicht entwickelt worden, vermutlich aufgrund des Fehlens prognostischer Parameter.

Es stellte sich daher die Aufgabe, ein neues Verfahren zur diagnostischen Beurteilung und Verlaufskontrolle sowie ein Arzneimittel zur Therapie von Schockzuständen beim Menschen zu finden.

Überraschender Weise ergab sich bei der Analyse des Bluts von Schockpatienten, daß einige von ihnen kurz nach Beginn oder im weiteren Verlauf der Schockkrankheit in erheblichem Umfang Cyclophiline im Serum ihres Blutes aufwiesen. Patienten, die zu irgend einem Zeitpunkt während der Schockerkrankung (zwischen dem ersten und fünfzehnten Tag) in deutlich meßbarem Umfang Cyclophiline im Serum aufwiesen, verstarben mit großer Regelmäßigkeit trotz der intensiven therapeutischen Bemühungen der behandelnden Ärzte. Patienten, die überlebten, zeigten bis zur Heilung zu keinem Zeitpunkt in meßbarem Umfang Cyclophiline in den zellfreien Anteilen ihres Blutes (Serum, Plasma, Plasmafraktionen etc.).

Nach heutigem Wissensstand enthalten alle Zellen des menschlichen und tierischen Organismus, aber auch viele Bakterien, Pflanzenzellen etc., eine besondere Gruppe von zytoplasmatischen Proteinen, die Cyclophiline (Galat, A., Eur J Biochem 1993, 216, 689). Sie dienen der Regulation der Proteinfaltung, der Proteinstabilität, Proteinfreisetzung, aber auch der Proteinbildung im Zusammenhang mit der Regulation der Funktion von Zellen im Normalzustand oder Streß, wie z.B. Infektionen, Transplantatabstoßungen etc. (Galat, s.o.) Cyclophiline sind sowohl im Zytoplasma als auch im Kern von eukaryoten Zellen festgestellt worden (Galat s.o.). Sie tauchen aber normalerweise nicht im extrazellularen Milieu auf, und es werden Ihnen keine wesentlichen extrazellularen Effekte zugeschrieben.

Aus der EP 0 326 067 - A2 ist bekannt, Cyclophilin in Säugetieren als antiinflammatorisches und immunmodulatorisches Mittel zur Behandlung von chronischen entzündlichen Reaktionen und Autoimmunerkrankungen einzusetzen. Die Aminosäuresequenz von Schweine- und Menschencyclophilin wird in dieser Anmeldung beschrieben.

In der EP 0 285 603 - A2 wird ein Verfahren zur Bestimmung von Cyclosporin-A im Blut beschrieben, wobei der zu untersuchenden Blutprobe eine definierte Menge an Cyclophilin und eine definierte Menge an markierten, an Cyclophilin bindender Substanz zugesetzt wird und der an das Cyclophilin gebundene Anteil an markierter Substanz durch Vergleich mit Standards der Pegel von Cyclosporin-A bzw. verwandter Immunsepresiva gemessen wird. Cyclosporin-A wird als Mittel zur Unterdrückung der immunologischen Abstoßungsreaktionen nach Organtransplantationen eingesetzt.

In der WO 83/00930 wird eine Schockbestimmung durch Unterdrückung der Lipoproteinlipase durch Endotoxin-induzierte Mediatoren beschrieben. Cyclophiline sind als Mediatoren nicht beschrieben.

In der WO 92/14455 wird ein Verfahren zur Kontrolle von abnormalen Konzentrationen von TNF-α in menschlichen Geweben beschrieben, die als Folge eines akuten septischen Schock auftreten können. Bevorzugte Mittel sind Phthalimidobipyridine. NF-α (Tumornekrosefaktor) wird der Gruppe der Cytokine zugerechnet. Daß auch Cyclophiline bei Schockzuständen eine Rolle spielen, wird in dieser Literaturstelle nicht erwähnt.

Die hier berichtete Beobachtung widerspricht dem Stand der Wissenschaft insofern als hier erstmals berichtet wird, daß im Zusammenhang mit der Schockkrankheit bei den Patienten, bei denen das Schockgeschehen letztendlich zum Tode führte, bereits lange vor ihrem Tod (Tage bis Wochen) erhebliche Mengen von Cyclophilinen im Blut nachweisbar waren. Obwohl die unterschiedlichsten Mediatoren bei z.B. septischem Schock gefunden wurden (de Boer, J.P., Immunopharmacology 1992, 24, 135), sind Cyclophiline nicht in Zusammenhang mit dem Schockgeschehen gebracht worden.

Das Auftreten von Cyclophilinen im Blutplasma oder Serum von Schockpatienten ist somit ein pathognomonisch ungünstiges Zeichen. Durch die Überwachung dieses Parameters im Serum lassen sich prognostische Aussagen treffen und die Therapie kontrollieren. Ein Abfall der Cyclophilinkonzentration ist als Zeichen eines möglichen Therapieerfolges zu werten, ein Anstieg als Warnsignal für einen bevorstehenden tödlichen Ausgang anzusehen. Bei experimentel hervorgerufenen Schockzuständen kann ferner die Cyclophilinkonzentration als Meßgröße für den Wirkungsnachweis von neuen Therapieformen verwendet werden.

Der hier beschriebene überraschende Befund legt darüber hinaus nahe, daß Cyclophiline als Mediatoren selbst negativ im Schockgeschehen aktiv werden.

Cyclophiline haben Ähnlichkeiten mit Zytokinen (Bang et al. Experientia 1993, 49, 533), einer Gruppe von Substanzen, die wesentlich an der Abwehrbereitschaft des menschlichen und tierischen Organismus beteiligt sind. Ihr unerwartetes Auftreten außerhalb der Zellgrenzen könnte daher eine massive Fehlregulation oder Umregulation der Abwehrbereitschaft des menschlichen Organismus provozieren, welche für die mittelbaren Folgen von Schockzuständen verantwortlich sind. Zur Therapie ist es deshalb erforderlich, die bei Schockpatienten auftretenden Cyclophiline zu inaktivieren, antagonisieren oder zu entfernen. Dazu stehen prinzipiell folgende Möglichkeiten zur Verfügung:
a) Blockade oder Antagonisierung der im Blutplasma auftretenden Cyclophiline durch blockierende und antagonisierende Pharmaka.
b) Blockade und Antagonisierung der auftretenden Cyclophiline durch biologische Wirkstoffe wie spezifische Antikörper, Antienzyme etc.
c) Entfernung und Zerstörung der im Blutplasma bzw. Serum auftretenden Cyclophiline durch physikalische und chemische Methoden wie Blutwäschen etc.

Der Nachweis der Cyclophiline im Serum kann mit den unterschiedlichen Methoden erfolgen, z.B. durch enzymologische Methoden, radiochemische Methoden, immunologische Methoden und klassische proteinbiochemische Methoden.

Die genannten diagnostischen Verfahren sind teilweise für den Nachweis der Cyclophiline in Zellen bekannt; weitere Verfahren stehen für andere Indikationen zur Verfügung und können auf die hier beschriebenen Situationen angepaßt werden. Beispielhaft kann die Cyclophilinkonzentration durch die Messung der Rotamaseaktivitat (Peptidyl-Prolin-cis-trans-lsomerase) gemäß folgender Beschreibung durchgeführt werden (H. Bang, Dissertation. Halle 1986, Wissenschaftsbereich Biochemie, Galat, s.o. und Fischer, G. et al., Biomed Biochim Acta 1984, 43, 1101):

Bestimmung von Isomerase Aktivitäten in Körperflüssigkeiten:
- das Analysesystem beruht auf dem Prinzip des gekoppelten spektroskopischen Enzymtestes d.h. die Reaktion eines Hilfsenzyms (Chymotrypsin) wird für die Detektion von Isomerasen eingesetzt.
- mittels einer Protease (Chymotrypsin) wird der konformationsspezifische Umsatz eines chromophormen Peptidsubstrates durchgeführt und spektroskopisch verfolgt.
- in einer ersten, sehr schnellen Reaktion spaltet die Protease selektiv die trans-Konformation,
- in einer zweiten, langsamen Phase wandelt sich die cis-Konformation unkatalysiert in die trans-Form um und kann danach durch die Protease gespalten werden.
- Isomerasen wie Cyclophiline beschleunigen selektiv diese cis-trans-Umwandlung.
- in zellfreien Körperflussigkeiten können damit Isomerasen über diesen spektroskopischen Nachweis analysiert werden,
- Nachweisgrenze: nM an Isomerase.

In ähnlicher Weise können auch mit Hilfe der vorhandenen spezifischen Antikörper gegen Cyclophiline, gemäß dem Stand der Technik, immunologische, radiochemische und andere Verfahren entwickelt werden.

Als therapeutische Verfahren ist eine Blockade des Cyclophilins mit z.B. Cyclosporin A prinzipiell klinisch anwendbar, ein in der Transplantationsmedizin, der Rheumatherapie, der Asthmatherapie und der Therapie z.B. der Psoriasis bereits verwendetes Mittel. Seine blockierende Wirkung auf Cyclophiline läßt sich in vitro belegen.

Beim Versuchstier (Maus) kann die Gabe von Cyclosporin A (CsA) das durch die Injektion von bakteriellen Lipopolysaccharid (LPS) oder TNFa (Tumornekrosefaktor a) ausgelösten letalen Schockzustand unterbinden (Das Leberversagen wurde in diesem Versuch durch den Nachweis lebertypischer Enzyme wie ALT (GPT), AST (GOT) und SDH (Sorbitdehydrogenase) quantifiziert. Die Ergebnisse sind in der Tabelle 1 wiedergegeben. Die Daten wurden 8 Stunden nach der Behandlung gemessen. Das CsA wurde in je 50 mg/kg i.v. 15 und 1 Stunde vor der Behandlung injiziert.

**Tabelle 1**

| **Protection by cyclosporin A against endotoxin- or TNFα-induced septic liver failure in galactosamine-sensitized mice** | | | |
|---|---|---|---|
| | **ALT [U/I]** | **AST [U/I]** | **SDH[U/I]** |
| unbehandelte Kontrolle | 40 ± 4 | 70 ± 8 | 40 ± 5 |
| LPS (5 µg/kg) | 1500 ± 1025 | 1044 ± 620 | 544 ± 340 |
| CsA + LPS | 86 ± 61 | 160 ± 83 | 25 ± 20 |
| TNF (15 µg/kg) | 2650 ± 730 | 2190 ± 920 | 1345 ± 370 |
| CsA + TNFα | 260 ± 134 | 210 ± 68 | 71 ± 49 |

### Ergebnisse klinischer Untersuchungen

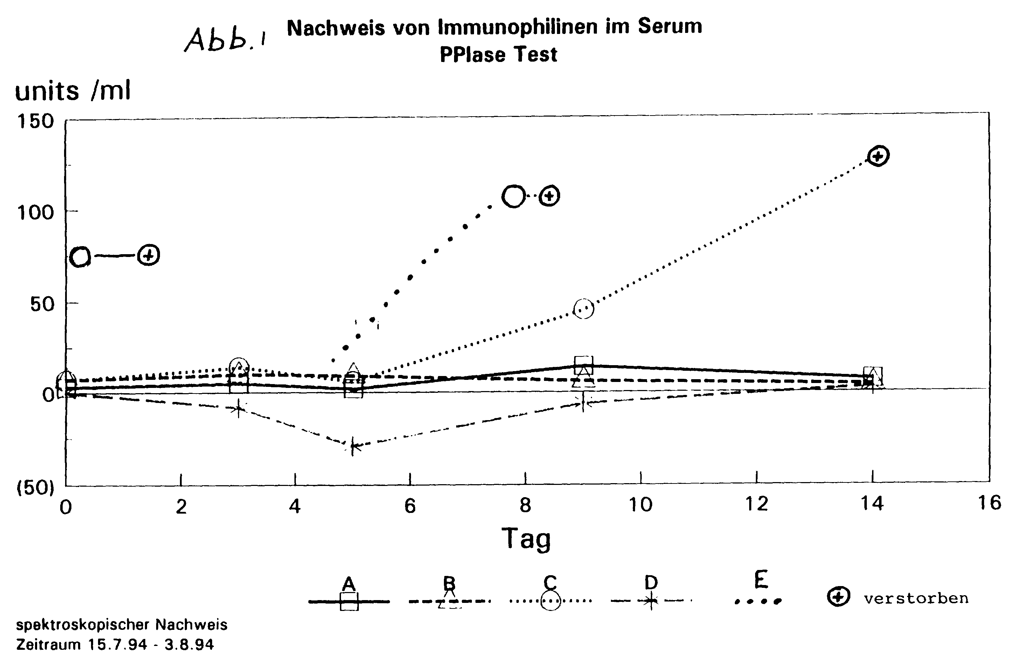

In der Abbildung sind Konzentrationsmessungen von Cyclophilinen im Serum von 6 Patienten mit Schockkrankheiten dargestellt. Es zeigte sich, daß bei Messung von Cyclophilinen, bei Anwendung der enzymatischen Nachweismethode (Peptidyl-Prolin-cis-trans-lsomerase, PPlase-Test) nur bei Patienten, die verstarben ansteigende oder erhöhte Mengen von Cyclophilinen im Plasma nachweisbar waren. Überraschenderweise traten diese intrazellularen Proteine bereits bis zu einer Woche vor dem späteren Exitus in deutlichem Umfang auf. Sie stiegen kurz vor dem Exitus erheblich an. Im Gegensatz dazu zeigten alle drei Patienten, die die Schockkrankheit überlebten, keinen meßbaren Anstieg der Cyclophilinkonzentration im Serum.

Cyclophilin Konzentrationen von über 50 U/ml scheinen daher auf einen letalen Ausgang der Schocksituation hinzuweisen, Konzentrationen von über 20 U/ml ein deutliches Warnsignal darzustellen.

### Quantifizierung und Bestimmungsmethodik:

Der PPlase-Test ist ein in Bezug auf die Molekülstruktur der eingesetzten Substrate und die proteolytische Empfindlichkeit der PPlasen in Grenzen variierbare Methode, mit der selbst PPlase-Aktivitäten in nur grob aufbereiteten biologischen Materialien quantitativ betimmt werden können. Für humanes rekombinates Cyclophilin liegt die noch nachweisbare Enzymkonzentration bei ca. 0,5 ng Cyclophilin pro ml Serum können detektiert werden. In grober Näherung entsprechen ca. 1 nM Cyclophilin einer Konzentrationsangabe von 1 U/ml. In Zusammenhang mit dem oben gesagten ergibt sich, daß eine Steigerung um 5 U/ml damit eine signifikante Erhöhung der CyclophilinKonzentration darstellt und klar von einem Wert 0 U/ml differenzierbar ist.

Des weiteren wurden im Serum von Sepsis Patienten nicht nur klar quantifizierbare Mengen von Cyclophilinen festgestellt (entspricht positiven Werten), sondern auch Modulatoren der Cyclophilinen. Im Falle einer negativen Angabe (-40 U/ml) handelt es sich um Inhibitoren. Diese Werte wurden ermittelt durch Überprüfung des Einflusses von Sepsis-Blut auf die extern zum PPlase-Test zugegebenen Cyclophiline.

Beispiel für die Bestimmung der Isomeraseaktivität
- Bereitstellung von zellfreien Serum;
- Einsatz eines UVNIS Spektrophotometers mit temperierbarem Küvettenhalter (Temperierung auf 10° C);
- 1 ml Hepes-Puffer werden mit 1 - 20 µl Serum für 30 sec. präinkubiert, danach werden 200 µl der Chymotrypsin-Lösung zugegeben und sofort mit 5 µl einer Substratlösung gestartet;
- die Reaktion wird kontinuierlich für 3 min bei einer Wellenlänge von 390 nm verfolgt;
- Die Geschwindigkeitskonstante der beobachteten Reaktion 1. Ordnung wird mit Hilfe eines Auswertungsprogrammes ermittelt und entspricht den in der Graphik verwendeten U/ml
- eine Messung mit Auswertung benötigt beim derzeitigen Verfahren ca. 10 min.

## Patentansprüche

1. Verfahren zum in vitro Nachweis von Schockzuständen und zur Therapiekontrolle von Schockpatienten, **dadurch gekennzeichnet**, daß die Konzentration von Cyclophilinen in Blutproben der Patienten mit Hilfe von
a) enzymologischen Methoden,
b) immunologischen Nachweismethoden mit Hilfe spezifischer Antikörper
c) peptidchemischen Verfahren,
d) radiochemischen Methoden
bestimmt wird und zur Bewertung des Schweregrades des Schocks dient.

2. Verfahren gemäß Anspruch 1, **dadurch gekennzeichnet**, daß
a) als enzymologische Methode die Rotamasen Aktivitätsmessung oder,
b) als immunologische Nachweismethode Radioimmunoassays, ELISA-Enzyme Linked Immuno-Sorben-Assay oder
c) als peptidchemisches Verfahren die Affinitätschromatographie oder
d) die Bindung radioaktiven Cyclosporins unter speziellen physikochemischen Reaktionsbedingungen eingesetzt wird.

3. Die Verwendung von Arzneistoffen und biologischen Wirkstoffen, welche die Konzentration der aktiven Cyclophiline im Blut von Schockpatienten vermindern, zur Herstellung von Arzneimitteln, zur Therapie des Schocks, insbesondere des septischen Schocks, des postoperativen Schocks und des toxischen Schocks.

4. Verwendung gemäß Anspruch 3, **dadurch gekennzeichnet**, daß man
a) Cyclosporine oder Cyclosporin verwandte, Cyclophiline inaktivierende Wirkstoffe,
b) spezifisch oder unspezifisch an Cyclophiline bindenden Pharmaka mit inaktivierenden Eigenschaften,
c) biologische Inaktivatoren der Cyclophiline, Antikörper, Antikörperfragmente, Cyclophiline zerstörende Enzyme,
einsetzt.

5. Verwendung von Cyclophiline bindenden oder zerstörenden Substanzen, zur Herstellung von Mitteln zur Blutwäsche im Blut von Schockpatienten.

6. Verwendung nach Anspruch 5, **dadurch gekennzeichnet**, daß diese Substanzen Cyclophilin-Antikörper, -Antikörperfragmente oder Cyclophilin zerstörende Enzyme sind.

## Claims

1. Process for the in vitro detection of states of shock and for the therapy control of shock patients, characterised in that the concentration of cyclophilines in blood samples of the patients is determined with the help of
a) enzymological methods,
b) immunological detection methods with the help of specific antibodies;
c) peptide-chemical processes;
d) radiochemical methods;
and serves for the evaluation of the degree of seriousness of the shock.

2. Process according to claim 1, characterised in that there is used
a) as enzymological method, the rotamase activity measurement or,
b) as immunological detection method, radio-immunoassays, ELISA enzyme linked immuno-sorban assay or
c) as peptide-chemical process, the affinity chromatography or
d) the binding of radio-active cyclosporin under special physicochemical reaction conditions.

3. The use of medicaments and biological active materials which reduce the concentration of the active cyclophilines in the blood of shock patients for the therapy of the shock, especially of the septic shock, of the post-operative shock and of the toxic shock.

4. Use according to claim 3, characterised in that one employs
a) cyclosporins or cyclosporin-related, cyclophiline-inactivating active materials,
b) pharmaceuticals with inactivating properties binding specifically or non-specifically to cyclophilines,
c) biological inactivators of the cyclophilines, antibodies, antibody fragments, cyclophiline-destroying enzymes.

5. Use of cyclophiline binding or destroying substances for the preparation of agents for the blood washing in the blood of shock patients.

6. Use according to claim 5, characterised in that these substances are cyclophiline antibodies, antibody fragments or cyclophiline-destroying enzymes.

## Revendications

1. Procédé de détection in vitro d'états de choc et de surveillance thérapeutique de patients en état de choc, caractérisé en ce qu'on détermine la concentration en cyclophilines dans des échantillons de sang provenant des patients à l'aide
a) de méthodes enzymologiques,
b) de méthodes de détection immunologiques à l'aide d'anticorps spécifiques,
c) de procédés utilisant la chimie des peptides,
d) de méthodes radiochimiques
et en ce que cette concentration sert à l'évaluation de l'importance du choc.

2. Procédé selon la revendication 1, caractérisé en ce qu'on utilise
a) à titre de méthode enzymologique, la mesure de l'activité de rotamase ou,
b) à titre de méthode de détection immunologique, des radioimmunodosages, le dosage ELISA (enzyme linked immuno-sorbent assay) ou
c) à titre de procédé de chimie des peptides, la chromatographie d'affinité ou
d) la liaison de la cyclosporine radioactive dans des conditions de réaction physico-chimiques spéciales.

3. Utilisation de médicaments et de principes actifs biologiques qui réduisent la concentration des cyclophilines actives dans le sang de patients en état de choc, pour la préparation de médicaments, pour la thérapie du choc, en particulier du choc septique, du choc postopératoire et du choc toxique.

4. Utilisation selon la revendication 3, caractérisé en ce qu'on utilise
a) des cyclosporines ou des principes actifs apparentés à la cyclosporine et inactivant les cyclophilines,
b) des agents pharmaceutiques se liant de manière spécifique ou non-spécifique aux cyclophilines et présentant des caractéristiques d'inactivation,
c) des inactivateurs biologiques des cyclophilines, des anticorps, des fragments d'anticorps, des enzymes détruisant les cyclophilines.

5. Utilisation de substances liant ou détruisant les cyclophilines pour la préparation d'agents d'épuration du sang, dans le sang de patients en état de choc.

6. Utilisation selon la revendication 5, caractérisée en ce que ces substances sont des anticorps de cyclophilines, des fragments d'anticorps de cyclophilines ou des enzymes détruisant les cyclophilines.
